# EUROPÄISCHE PATENTSCHRIFT

(11) **EP 1 432 746 B1**
(45) Veröffentlichungstag und Bekanntmachung des Hinweises auf die Patenterteilung: **07.02.2007**
(21) Anmeldenummer: 01989525.9
(22) Anmeldetag: 28.11.2001
(51) Int. Cl.: A61K 8/81, A61K 8/86, A61K 8/91, A61Q 15/00, A61Q 19/00, C08F 220/58, C08F 8/28, C08F 265/00, C08F 290/06, C08F 291/00, C08L 51/00

(54) **DEODORANTIEN UND ANTIPERSPIRANTIEN**
DEODORANTS AND ANTI-PERSPIRANTS
DEODORANTS ET ANTI-TRANSPIRANTS

(30) Priorität: 01.12.2000 DE 10059823
(43) Veröffentlichungstag der Anmeldung: 30.06.2004
(73) Patentinhaber: Clariant Produkte (Deutschland) GmbH, 65929 Frankfurt am Main (DE)
(72) Erfinder: LÖFFLER, Matthias, 65527 Niedernhausen (DE); MORSCHHÄUSER, Roman, 55122 Mainz (DE)
(86) Internationale Anmeldenummer: PCT/EP2001/013863
(87) Internationale Veröffentlichungsnummer: WO 2002/043687

(56) Entgegenhaltungen:
- EP-A- 0 510 246
- EP-A- 0 522 756
- EP-A- 0 603 019
- EP-A- 0 642 781
- EP-A- 0 815 828
- WO-A-00/12588
- WO-A-01/62214
- DATABASE CAPLUS [Online] CHEMICAL ABSTRACTS SERVICE, COLUMBUS, OHIO, US; MIYAZAWA,KAZUYUKI ET AL: "Sulfo group-containing polysiloxane block copolymers and cosmetics containing them" retrieved from STN Database accession no. 2001:736921 XP002204070 & JP 2001 278982 A (SHISEIDO CO. LTD., JAPAN) 10. Oktober 2001 (2001-10-10)

## Beschreibung

Die vorliegende Erfindung betrifft Deodorantien und Antiperspirantien, enthaltend kammförmige Copolymere auf Basis von Acryloyldimethyltaurinsäure.

Deodorantien und Antiperspirantien werden in unterschiedlichen Formen, wie Gele, Sticks, Cremes, Sprays oder Puder dargeboten. Kosmetische Deodorierungsmittel dienen dazu, Körpergeruch zu beseitigen, der entsteht, wenn der an sich geruchlose Schweiß durch Mikroorganismen zersetzt wird. Den üblichen kosmetischen Deodorantien liegen unterschiedliche Wirkprinzipien zugrunde.
In sogenannten Antiperspirantien kann durch Adstringenzien- beispielsweise Aluminiumsalze wie Aluminiumhydroxychlorid - die Bildung des Schweißes reduziert werden. Durch die Verwendung antimikrobieller Stoffe in kosmetischen Deodorantien kann die Bakterienflora auf der Haut reduziert werden. Dabei sollten im Idealfall nur die Geruch verursachenden Mikroorganismen wirksam reduziert werden. Auch die Kombination von Adstringenzien mit antimikrobiell wirksamen Stoffen ist gebräuchlich.
Insbesondere antiperspirierende Wirkstoffe sind häufig mit gängigen Bestandteilen deodorierender kosmetischer und pharmazeutischer Präparate nicht kompatibel. So werden deodorierende Stifte in der Regel aus Seifen-Glykol-Gelen gebildet, da niedere Glykole und Glycerin in Gegenwart von Natriumstearat klare, transparente Gele bilden können, welche zusätzlich Alkohol und Wasser aufnehmen können. Aufgrund der Alkalinität der Seifen können saure Wirkstoffe, beispielsweise Aluminiumhydroxychlorid in solche Träger nicht eingearbeitet werden.

Wegen dieser Nachteile wurden auch seifenfreie Deodorantien und Antiperspiraritien entwickelt, z.B. auf Basis flüssiger Öle (z.B. Paraffinöle, Ricinusöl, Isopropylpalmitat), die mit halbfesten Bestandteilen (z.B. Vaseline, Lanolin), festen Bestandteilen (z.B. Bienenwachs, Ceresin, mikrokristallinen Wachse bzw. Ozokerit) und/ oder hochschmelzenden Wachsen (z.B. Carnaubawachs, Candelillwachs) verdickt sind. Nachteilig ist, dass wasserlösliche Wirkstoffe häufig nicht genug fettlöslich sind und folglich nur unzureichend in kosmetische Grundlagen eingebaut werden können. Andererseits ist ein gewisser Wassergehalt durchaus erwünscht, um die Verträglichkeit der Mittel mit der menschlichen Haut oder um die Löslichkeit von salzartigen Inhaltsstoffen zu verbessern. Als flüssige Öle eignen sich Silikonöle, die ein sehr angenehmes Hautgefühl der Mittel ergeben. Diese sind jedoch sehr teuer und weisen Nachteile bei der stabilen Einarbeitung wasserlöslicher Wirkstoffe auf und sind in Mitteln mit höheren Wassergehalten schwer formulierbar.

Überraschend wurde nun gefunden, dass sich eine neue Klasse von Kammpolymeren auf Basis von Acryloyldimethyltaurinsäure (AMPS) - die sich sowohl als Verdicker, Konsistenzgeber, Emulgator, Solubilisator, Dispergator, Gleitmittel, Haftmittel, Conditioner und/oder Stabilisator eignen - hervorragend zur Formulierung von Deodorantien und Antiperspirantien eignen.

Gegenstand der Erfindung sind Deodorantien und Antiperspirantien, enthaltend mindestens ein Copolymer, erhältlich durch radikalische Copolymerisation von
A) Acryloyldimethyltaurinsäure und/oder Acryloyldimethyliauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) einer oder mehreren mindestens monofunktionellen, zur radikalischen Polymerisation befähigten, siliziumhaltigen Komponente(n), wobei mindestens eine siliziumhaltige Komponente eine Verbindung der Formel (I)

   R¹-Z-[(Si(R³R⁴)-O-)_{w}-(Si(R⁵R⁶)-O)ₓ-]-R² (I)

   ist, wobei
   R¹ einen Vinyl-, Allyl-, Methallyl-, Methylvinyl-, Acryl-(CH₂=CH-CO-), Methacryl-(CH₂=C[CH_{3]}-CO-), Crotonyl-, Senecionyl-, Itaconyl-, Maleinyl-, Fumaryl- oder Styrylrest darstellt,
   Z eine chemische Brücke, bevorzugt -O-, -((C₁-C₅₀)Alkylen)-, -((C₆-C₃₀) Arylen)-, -((C₅-C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann, -((C₁-C₁₀)Alkyl)-(Si(OCH₃)₂)- und -(Si(OCH₃)₂)-, darstellt,
   R³, R⁴, R⁵ und R⁶ unabhängig voneinander -CH₃, -O-CH₃, -C₆H₅ oder -O-C₆H₅ bedeuten,
   w und x stöchiometrische Koeffizienten repräsentieren, die unabhängig voneinander 0 bis 500, bevorzugt 10 bis 250, betragen, wobei entweder w oder x größer Null sein muss und wobei die Verteilung der Wiederholungseinheiten über die Kette hinweg nicht nur rein statistisch, sondern auch blockartig, alternierend oder gradientenartig sein kann,
   R² einen ungesättigten aliphatischen, cycloaliphatischen, arylaliphatischen oder aromatischen Rest mit jeweils 1 bis 50 C-Atomen, vorzugsweise einen olefinischen (C₁-C₅₀)-Kohlenwasserstoffrest symbolisiert (linear oder verzweigt) oder für die Struktureinheit [-Z-R¹] steht, wobei Z und R¹ die oben genannten Bedeutungen haben,
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation gegebenenfalls in Gegenwart mindestens eines polymeren Additivs mit zahlenmittleren Molekulargewichten von 200 g/mol bis 10⁹ g/mol erfolgt.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure (Acrylamidopropyl-2-methyl-2-sulfonsäure) handeln. Bevorzugt werden die Li⁺-, Na⁺-, K⁺-, Mg⁺⁺-, Ca⁺⁺-, Al⁺⁺⁺- und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handelt. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80%.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.
Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.
Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.
Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammonium-reste, wobei es sich bei den Alkylsubstituenten der Amine unabhängig voneinander um (C₁-C₂₂)-Alkylreste oder (C₂-C₁₀)-Hydroxyalkylreste handelt. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100 % betragen.
Als Comonomere B) weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethylacrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester]; N,N-Dimethylaminomethacrylat; Diethylamino-methylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.
Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden; dass unter den kationischen Comonomeren C) auch solche zu verstehen sind, die die kationische Ladung in Form einer betainischen, zwitterionischen, oder amphoteren Struktur tragen.
Comonomere C) im Sinne der Erfindung sind auch aminofunktionalisierte Precursor, die durch polymeranaloge Reaktionen in Ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphoteren Derivate überführt werden können.

Besonders bevorzugt als Comonomere C) sind
Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain.

Der Gewichtsanteil der Comonomeren C) kann, bezogen auf die Gesamtmasse der Copolymere, 0,1 bis 99,8 Gew.-%, bevorzugt 0,5 bis 30 Gew.-% und besonders bevorzugt 1 bis 20 Gew.-% betragen.

Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Wenn R² in den Verbindungen der Formel (I) ein Element der Gruppe [-Z-R¹] darstellt, handelt es sich um difunktionelle, Monomere, die zur Vemetzung der entstehenden Polymerstrukturen herangezogen werden können.
Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Komponenten sind die folgenden acrylisch- oder methacrylisch modifizierten silikonhaltigen Komponenten:

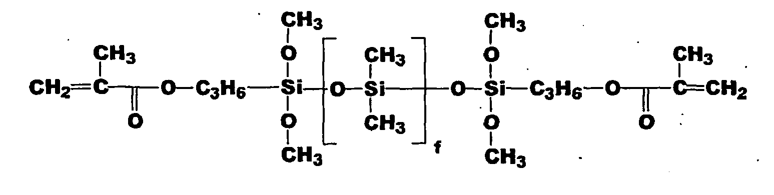

Methacryloxyproplydimethylsilyl endgeblockte Polydimethylsiloxane (f=2 bis 500)

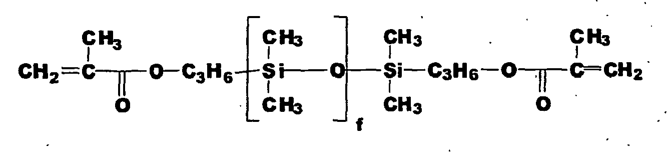

Methacryloxypropyl endgeblockte Polydimethylsiloxane (f= 2 bis 500 bis)

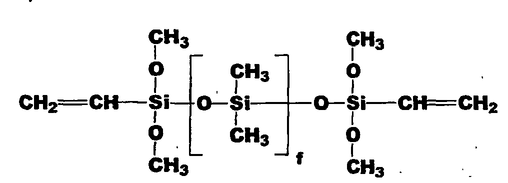

Vinyldimethoxysilyl endgeblockte Polydimethylsiloxane (f = 2-500)

Bezogen auf die Gesamtmasse der Copolymere kann der Gehalt an siliziumhaltigen Komponenten bis 99,8 Gew.-%, bevorzugt 0,5 bis 30 Gew.-%, insbesondere bevorzugt 1 bis 20 Gew.-%, betragen.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch muttibtock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, dass monofunktionelle Vertreter zur Bildung kammförmiger Strukturen führen, wohingegen di-, tri-, oder polyfunktionelle Komponenten E) zu zumindest teilvernetzten Strukturen führen.

Bevorzugte fluorhaltige Komponenten E) sind solche gemäß Formel (II).

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest, besonders bevorzugt einen Acryl- und Methacrylrest, dar.
Zur Anbindung der fluorhaltigen Gruppierung an die reaktive Endgruppe R¹ ist eine geeignete chemische Brücke Y erforderlich. Bevorzugte Brücken Y sind -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann.
Bei r und s handelt es sich um stöchiometrische Koeffizienten, die unabhängig voneinander Zahlen von 0 bis 200 bedeuten.

Bevorzugte fluorhaltige Komponenten E) gemäß Formel (II) sind
Perfluorhexylethanol-methacrylat,
Perfluorhexoylpropanol-methacrylat,
Perfluoroctyethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctyethanolyl-poly-[ethylglykol-blockco-propylenglycolether]-methacrylat,
Perfluoroctylpropanolyl-polypropylen-glycolether-methacrylat.

Bezogen auf die Gesamtmasse des Copolymeren kann der Gehalt an fluorhaltigen Komponenten bis 99,9 Gew.-%, bevorzugt 0,5 bis 30 Gew.-%, insbesondere bevorzugt 1 bis 20 Gew.-%, betragen.

Bei den Makromonomeren F) handelt sich um mindestens einfach olefinisch funktionalisierte Polymere mit einer oder mehreren diskreten Wiederholungseinheiten und einem zahlenmittleren Molekulargewicht größer oder gleich 200 g/mol. Bei der Copolymerisation können auch Mischungen chemisch unterschiedlicher Makromonomere F) eingesetzt werden. Bei den Makromonomeren handelt es sich um polymere Strukturen, die aus einer oder mehreren Wiederholungseinheit(en) aufgebaut sind und eine für Polymere charakteristische Molekulargewichtsverteilung aufweisen.
Bevorzugt als Makromonomere F) sind Verbindungen gemäß Formel (III).

R¹-Y-[(A)ᵥ-(B)_{w}-(C)ₓ-(D)_{z}]-R² (III)

R¹ stellt eine polymerisationsfähige Funktion aus der Gruppe der vinylisch ungesättigten Verbindungen dar, die zum Aufbau polymerer Strukturen auf radikalischem Wege geeignet sind. Bevorzugt stellt R¹ einen Viriyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.
Zur Anbindung der Polymerkette an die reaktive Endgruppe ist eine geeignete verbrückende Gruppe Y erforderlich. Bevorzugte Brücken Y sind -O-, -C(O)-, -C(O)-O-, -S-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)-, besonders bevorzugt -O-.
Der polymere Mittelteil des Makromonomeren wird durch die diskreten Wiederholungseinheiten A, B, C und D repräsentiert. Bevorzugte Wiederholungseinheiten A,B,C und D leiten sich ab von Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid.

Die Indizes v, w, x und z in Formel (III) repräsentieren die stöchiometrische Koeffizienten betreffend die Wiederholungseinheiten A, B, C und D. v, w, x und z betragen unabhängig voneinander 0 bis 500, bevorzugt 1 bis 30, wobei die Summe der vier Koeffizienten im Mittel ≥ 1 sein muss.
Die Verteilung der Wiederholungseinheiten über die Makromonomerkette kann statistisch, blockartig, alternierend oder gradientenartig sein.
R² bedeutet einen linearen oder verzweigten aliphatischen, olefinischen, cycloaliphatischen, arylaliphatischen oder aromatischen (C₁-C₅₀)-Kohlenwasserstoffrest, OH, -NH₂, -N(CH₃)₂ oder ist gleich der Struktureinheit [-Y-R¹].
Im Falle von R² gleich [-Y-R¹] handelt es sich um difunktionelle Makromonomere, die zur Vernetzung der Copolymere geeignet sind.

Besonders bevorzugt als Makromonomere F) sind acrylisch- oder methacrylisch monofunktionalisierte Alkylethoxylate gemäß Formel (IV).

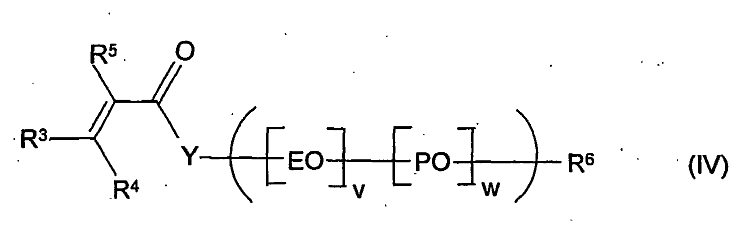

R₃, R₄, R₅ und R₆ bedeuten unabhängig voneinander Wasserstoff oder n-aliphatische, iso-aliphatische, olefinische, cycloaliphatische, arylaliphatische oder aromatische (C₁-C₃₀)-Kohlenwasserstoffreste.
Bevorzugt sind R₃ und R₄ gleich H oder -CH₃, besonders bevorzugt H; R₅ ist gleich H oder -CH₃; und R₆ ist gleich einem n-aliphatischen, iso-aliphatischen, olefinischen, cycloaliphatischen, arylaliphatischen oder aromatischen (C₁-C₃₀)-Kohlenwasserstoffrest.
v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥ 1 sein muss. Die Verteilung der EO- und PO-Einheiten über die Makromonomerkette kann statistisch, blockartig, alternierend oder gradientenartig sein. Y steht für die obengenannten Brücken.

Weiterhin insbesondere bevorzugte Makromonomeren F) haben die folgende Struktur gemäß Formel (IV):

| Bezeichnung | R³ | R⁴ | R⁵ | R⁶ | v | w |
|---|---|---|---|---|---|---|
| ^{®}LA-030-methyacrylat | H | H | -CH₃ | -Lauryl | 3 | 0 |
| ^{®}LA-070-methacrylat | H | H | -CH₃ | -Lauryl | 7 | 0 |
| ^{®}LA-200-methacrylat | H | H | -CH₃ | -Lauryl | 20 | 0 |
| ^{®}LA-250-methacrylat | H | H | -CH₃ | -Lauryl | 25 | 0 |
| ^{®}T-080-methyacrylat | H | H | -CH₃ | -Talk | 8 | 0 |
| ^{®}T-080-acrylat | H | H | H | -Talk | 8 | 0 |
| ^{®}T-250-methyacrylat | H | H | -CH₃ | -Talk | 25 | 0 |
| ^{®}T-250-crotonat | -CH₃ | H | -CH₃ | -Talk | 25 | 0 |
| ^{®}OC-030-methacrylat | H | H | -CH₃ | -Octyl | 3 | 0 |
| ^{®}OC-105-methacrylat | H | H | -CH₃ | -Octyl | 10 | 5 |
| ^{®}Behenyl-010-methyaryl | H | H | H | -Behenyl | 10 | 0 |
| ^{®}Behenyl-020-methyaryl | H | H | H | -Behenyl | 20 | 0 |
| ^{®}Behenyl-010-senecionyl | -CH₃ | -CH₃ | H | -Behenyl | 10 | 0 |
| ^{®}PEG-440-diacrylat | H | H | H | -Acryl | 10 | 0 |
| ^{®}B-11-50-methacrylat | H | H | -CH₃ | -Butyl | 17 | 13 |
| ^{®}MPEG-750-methacrylat | H | H | -CH₃ | -Methyl | 18 | 0 |
| ^{®}P-010-acrylat | H | H | H | -Phenyl | 10 | 0 |
| ^{®}O-050-acrylat | H | H | H | -Oleyl | 5 | 0 |

Weiterhin als Makromonomere F) insbesondere geeignet sind Ester der (Meth)acrylsäure mit
(C₁₀-C₁₈)-Fettalkoholpolyglykolethern mit 8 EO-Einheiten (Genapol^{®} C-080)
C₁₁-Oxoalkoholpolyglykolethern mit 8 EO-Einheiten (Genapol^{®} UD-080)
(C₁₂-C₁₄)-Fettalkoholpolyglykolethern mit 7 EO-Einheiten (Genapol^{®} LA-070)
(C₁₂-C₁₄)-Fettalkoholpolyglykolethern mit 11 EO-Einheiten (Genapol^{®} LA-110)
(C₁₆-C₁₈)-Fettalkoholpolyglykolethem mit 8-EO-Einheiten (Genapol^{®} T-080)
(C₁₆-C₁₈)-Fettalkoholpolyglykolethern mit 15 EO-Einheiten (Gemipol^{®} T-150)
(C₁₆-C₁₈)-Fettalkoholpolyglykolethern mit 11 EO-Einheiten (Genapol^{®} T-110)
(C₁₆-C₁₈)-Fettatkoholpotygtycotethern mit 20 EO-Einheiten (Genapol^{®} T-200)
(C₁₆-C₁₈)-Fettalkoholpolyglykolethern mit 25 EO-Einheiten (Genapol^{®} T-250)
(C₁₈-C₂₂)-Fettalkoholpolyglykolethern mit 25 EO-Einheiten und/oder
iso-(C₁₆-C₁₈)-Fettalkoholpolyglykolethern mit 25 EO-Einheiten.
Bei den Genapol^{®}-Typen handelt es sich um Produkte der Firma Clariant, GmbH.

Bevorzugt beträgt das Molekulargewicht der Makromonomeren F) 200 g/mol bis 10⁶ g/mol, besonders bevorzugt 150 bis 10⁴ g/mol und insbesondere bevorzugt 200 bis 5000 g/mol.

Bezogen auf die Gesamtmasse der Copolymere können geeignete Makromonomere bis zu 99,8 Gew.-% eingesetzt werden. Bevorzugt finden die Bereiche 0,5 bis 30 Gew.-% und 70 bis 99,5 Gew.-% Anwendung. Besonders bevorzugt sind Anteile von 1 bis 20 Gew.-% und 75 bis 95 Gew.-%.

Bevorzugt als Copolymere sind solche, die durch Copolymerisation mindestens der der Komponenten A), C) und D) erhältlich sind.

Weiterhin bevorzugt als Copolymere sind solche, die durch Copolymerisation mindestens der Komponenten A), D) und F) erhältlich sind.

Weiterhin bevorzugt als Copolymere sind solche, die durch Copolymerisation mindestens der Komponenten A) und D) erhältlich sind.

In einer bevorzugten Ausführungsform wird die Copolymerisation in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.
Die Additive G) bzw. deren Mischungen müssen lediglich ganz oder teilweise im gewählten Polymerisationsmedium löslich sein. Während des eigentlichen Polymerisationsschrittes hat das Additiv G) mehrere Funktionen. Einerseits verhindert es im eigentlichen Polymerisationsschritt die Bildung übervernetzter Polymeranteile im sich bildenden Copolymerisat und andererseits wird das Additiv G) gemäß dem allgemein bekannten Mechanismus der Pfropf-Copolymerisation statistisch von aktiven Radikalen angegriffen. Dies führt dazu, dass je nach Additiv G) mehr oder weniger große Anteile davon in die Copolymere eingebaut werden. Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.
Bevorzugt als Additive G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen.
Besonders bevorzugt als Additive G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole.
Insbesondere bevorzugt als Additive G) sind Polyvinylpyrrolidone (z.B. Luviskol K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können.

Das Molekulargewicht der Additive G) beträgt bevorzugt 10² bis 10⁷ g/mol, besonders bevorzugt 0,5*10⁴ bis 10⁶ g/mol.

Die Einsatzmenge des polymeren Additivs G) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.
Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylengiykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.
Insbesondere bevorzugt als Vernetzer ist Trimethylolpropantriacrylat (TMPTA).

Der Gewichtsanteil an vernetzenden Comonomeren, bezogen auf die Gesamtmasse der Copolymere, beträgt bevorzugt bis 20 Gew.-%, besonders bevorzugt 0,05 bis 10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodiisobutyronitril (AIBN), verwendet werden.
Ebenfalls geeignet sind anorganische Peroxyverbindungen, wie z.B. (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.
Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden Wasser und niedere, tertiäre Alkohole oder Kohlenwasserstoffe mit 3 bis 30 C-Atomen. In einer besonders bevorzugten Ausführungsweise wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander mischbaren Solventien ein (z.B. Wasser/Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).

Bevorzugt eignet sich die Fällungspolymerisation, besonders bevorzugt die Fällungspolymerisation in tert.-Butanol.

Die nachfolgende Auflistung zeigt 67 Copolymere, die für die Formulierung der erfindungsgemäßen Mittel besonders geeignet sind. Die verschiedenen Copolymere Nr. 1 bis Nr. 67 sind gemäß den folgenden Herstellverfahren 1, 2, 3 und 4 erhältlich. Die Copolymere Nr. 28 bis 40, 53, 60, 61 und 63 sind Copolymere, die durch radikalische Copolymerisation von zumindest Komponente A) und Komponente D) erhältlich sind. Die Copolymere Nr. 1 bis 27, 41 bis 52, 54 bis 59, 62 und 64 bis 67 stellen demgegenüber keine Copolymere dar, die durch radikalische Copolymerisation von zumindest Komponente A) und Komponente D) erhältlich sind.

### Verfahren 1:

Diese Polymere sind nach dem Fällungsverfahren in tert. Butanol herstellbar. Dabei wurden die Monomere in t-Butanol vorgelegt, die Reaktionsmischung inertisiert und anschließend die Reaktion nach Anheizen auf 60°C durch Zugabe des entsprechenden t-Butanol löslichen Initiators (bevorzugt Dilauroylperoxid) gestartet. Die Polymere werden nach beendeter Reaktion (2 Stunden) durch Absaugen des Lösungsmittels und durch anschließende Vakuumtrocknung isoliert.

### Verfahren 2:

Diese Polymere sind nach dem Gelpolymerisationsverfahren in Wasser herstellbar. Dabei werden die Monomere in Wasser gelöst, die Reaktionsmischung inertisiert und anschließend die Reaktion nach Anheizen auf 65°C durch Zugabe von geeigneten Initiatoren- oder Initiatorsystemen (bevorzugt Na₂S₂O₈) gestartet. Die Polymergele werden anschließend zerkleinert und nach Trocknung die Polymere isoliert.

### Verfahren 3:

Diese Polymere sind nach dem Emulsionsverfahren in Wasser herstellbar. Dabei werden die Monomere in einer Mischung aus Wasser/organ. Lösungsmittel (bevorzugt Cyclohexan) unter Verwendung eines Emulgators emulgiert, die Reaktionsmischung mittels N₂ inertisiert und anschließend die Reaktion nach Anheizen auf 80°C durch Zugabe von geeigneten Initiatoren- oder Initiatorsystemen (bevorzugt Na₂S₂O₈) gestartet. Die Polymeremulsionen werden anschließend eingedampft (Cyclohexan fungiert als Schlepper für Wasser) und dadurch die Polymere isoliert.

### Verfahren 4:

Diese Polymere sind nach dem Lösungsverfahren in organischen Lösungsmitteln (bevorzugt Toluol, z.B. auch tert. Alkohole) herstellbar. Dabei werden die Monomere im Lösungsmittel vorgelegt, die Reaktionsmischung inertisiert und anschließend die Reaktion nach Anheizen auf 70°C durch Zugabe von geeigneten Initiatoren- oder Initiatorsystemen (bevorzugt Dilauroylperoxid) gestartet. Die Polymere werden durch Abdampfen des Lösungsmittels und durch anschließende Vakuumtrocknung isoliert.

Polymere mit hydrophoben Seitenketten, unvernetzt

| Nr. | Zusammensetzung | | Herstellverfahren |
|---|---|---|---|
| 1 | 95 g AMPS | 5 g Genapol T-080 | 1 |
| 2 | 90 g AMPS | 10 g Genapol T-080 | 1 |
| 3 | 85 g AMPS | 15 g Genapol T-080 | 1 |
| 4 | 80 g AMPS | 20 g Genapol T-080 | 1 |
| 5 | 70 g AMPS | 30 g Genapol T-080 | 1 |
| 6 | 50 g AMPS | 50 g Genapol T-080 | 3 |
| 7 | 40 g AMPS | 60 g Genapol T-080 | 3 |
| 8 | 30 g AMPS | 70 g Genapol T-080 | 3 |
| 9 | 20 g AMPS | 80 g Genapol T-080 | 3 |
| 10 | 60 g AMPS | 60 g BB10 | 4 |
| 11 | 80 g AMPS | 20 g BB10 | 4 |
| 12 | 90 g AMPS | 10 g BB10 | 3 |
| 13 | 80 g AMPS | 20 g BB10 | 1 |
| 14 | 80 g AMPS | 20 g Genapol LA040 | 1 |

Polymere mit hydrophoben Seitenketten, vernetzt

| Nr. | Zusammensetzung | | | Herstellverfahren |
|---|---|---|---|---|
| 15 | 80 g AMPS | 20 g Genapol LA040 | 0.6g AMA | 1 |
| 16 | 80 g AMPS | 20 g Genapol LA040 | 0,8 AMA | 1 |
| 17 | 80 g AMPS | 20 g Genapol LA040 | 1,0 g AMA | 1 |
| 18 | 628,73 g AMPS 120,45 g Genapol T-250 6,5 g TMPTA | | | 2 |
| 19 | 60 g AMPS | 40 g BB10 | 1,9 g TMPTA | 4 |
| 20 | 80 g AMPS | 20 g BB10 | 1,4 g TMPTA | 4 |
| 21 | 90 g AMPS | 10 g BB10 | 1,9 g TMPTA | 4 |
| 22 | 80 g AMPS | 20 g BB10 | 1,9 g TMPTA | 4 |
| 23 | 60 g AMPS | 40 g BB10 | 1,4 g TMPTA | 4 |

Polymere mit hydrophoben Seitenketten, vernetzt, gepfropft

| Nr. | Zusammensetzung | | | | Herstellverfahren |
|---|---|---|---|---|---|
| 24 | 95 g AMPS | 5 g BB10, | 1,9 g TMPTA, | 1 g Poly-NVP | 1 |
| 25 | 90 g AMPS | 10 g BB10, | 1,9 g TMPTA, | 1 g Poly-NVP | 1 |
| 26 | 85 g AMPS | 15 g BB10, | 1;9 g TMPTA, | 1 g Poly-NVP | 1 |
| 27 | 90 g AMPS | 10 g BB10, | 1,9 g TMPTA, | 1 g Poly-NVP | 1 |

Polymere mit siliziumhaltigen Gruppen, unvernetzt

| Nr. | Zusammensetzung | | Herstellverfahren |
|---|---|---|---|
| 28 | 80 g AMPS, | 20 g Silvet 867 | 1 |
| 29 | 80 g AMPS, | 50 g Silvet 867 | 4 |

Polymere mit siliziumhaltigen Gruppen, vernetzt

| Nr. | Zusammensetzung | | | Herstellverfahren |
|---|---|---|---|---|
| 30 | 80 g AMPS, | 20 g Silvet 867, | 0,5 g MBA | 4 |
| 31 | 80 g AMPS, | 20 g Silvet 867, | 1,0 g MBA | 1 |
| 32 | 60 g AMPS, | 40 g Y-12867, | 0,95 g AMA | 1 |
| 33 | 80 g AMPS, | 20 g Y-12867, | 0,95g AMA | 1 |
| 34 | 90 g AMPS, | 10 g Y-12867, | 0,95 g AMA | 1 |
| 35 | 60 g AMPS, | 40 g Silvet 7280, | 0,95 g AMA | 1 |
| 36 | 80 g AMPS, | 20 g Silvet 7280, | 0,95 g AMA | 1 |
| 37 | 90 g AMPS, | 10 g Silvet 7280, | 0,95 g AMA | 1 |
| 38 | 60 g AMPS, | 40 g Silvet 7608, | 0,95 g AMA | 1 |
| 39 | 80 g AMPS, | 20 g Silvet 7608, | 0,95 g AMA | 1 |
| 40 | 90 g AMPS, | 10 g Silvet 7608, | 0,95 g AMA | 1 |

Polymere mit hydrophoben Seitenketten und kationischen Gruppen, unvernetzt

| Nr. | Zusammensetzung | | | Herstellverfahren |
|---|---|---|---|---|
| 41 | 87,5 g AMPS, | 7,5 g Genapol T-110, | 5 g DADMAC | 2 |
| 42 | 40 g AMPS, | 10 g Genapol T110, | 45 g Methacrylamid | 2 |
| 43 | 55 g AMPS, | 40 g Genapol LA040, | 5 g Quat | 1 |
| 44 | 75 g AMPS, | 10 g BB10, | 6,7 g Quat | 1 |

Polymere mit hydrophoben Seitenketten und kationischen Gruppen, vernetzt

| Nr. | Zusammensetzung | | | | Herstellverfahren |
|---|---|---|---|---|---|
| 45 | 60 g AMPS, | 20 g Genapol T-80, | 10 g Quat, | 10 g HEMA | 1 |
| 46 | 75 g AMPS, | 20 g GenapolT-250, | 5 g Quat, | 1,4 g TMPTA | 1 |
| 47 | 75 g AMPS, | 20 g GenapolT-250, | 10 g Quat, | 1,4 g TMPTA | 1 |
| 48 | 75 g AMPS, | 20 g GenapolT-250, | 20 g Quat, | 1,4 g TMPTA | 1 |

Polymere mit fluorhaltigen Gruppen

| Nr. | Zusammensetzung | Herstellverfahren |
|---|---|---|
| 49 | 94 g AMPS, 2,02 g Fluowet AC 600 | 1 |
| 50 | 80 g AMPS, 20 g Perfluoroctylpolyethylenglykolmethacrylat, 1 g Span 80 | 3 |

Polymere mit fluorhaltigen Gruppen, gepfropft

| Nr. | Zusammensetzung | | Herstellverfahren |
|---|---|---|---|
| 51 | 80 g AMPS, | 10 g Fluowet AC 600, 5 g Poly-NVP | 1 |
| 52 | 70 g AMPS, 5 g Poly-NVP | 8 g Perfluoroctylethyloxyglycerinmethacrylat, | 4 |

Multifunktionelle Polymere

| Nr. | Zusammensetzung | Herstellverfahren |
|---|---|---|
| 53 | 80 g AMPS, 10 g Genapol LA070, 10 g Silvet 7608, | 1 |
| | 1,8 g TMPTA | |
| 54 | 70 g AMPS, 5 g N-Vinylpyrrolidon, 15 g Genapol T-250 | 4 |
| | methacrylat, 10 g Quat, 10 g Poly-NVP | |
| 55 | 80 g AMPS, 5 g N-Vinylformamid, 5 g Genapol O-150- | 2 |
| | methacrylat, 10 g DADMAC, 1,8 g TMPTA, 8 g Poly-N- | |
| | Vinylformamid | |
| 56 | 70 g AMPS, 5 g N-Vinylpyrrolidon, 15 g Genapol T- | 1 |
| | 250-methacrylat, 10 g Quat, 10 g Poly-NVP | |
| 57 | 60 g AMPS, 10 g Genapol-BE-020-methacrylat, | 1 |
| | 10 g Genapol T-250-acrylat, 20 g Quat, 1 g Span 80 | |
| 58 | 60 g AMPS, 20 g MPEG-750-methacrylat, | 1 |
| | 10 g Methacryloxypyldimethicon, | |
| | 10 g Perfluorooctylpolyethylenglycol-methacrylat, | |
| | 10 g Poly[N-vinylcaprolacton-co-acrylsäure] (10/90) | |
| 59 | 80 g AMPS, 5 g N-Vinylformamid, 5 g Genapol O-150- | 1 |
| | methacrylat, 10gDADMAC, 1,8 g TMPTA | |
| 60 | 70 g AMPS, 10 g Genapol T-250-acrylat, 5 g N-Methyl- | 1 |
| | 4-vinylpyridiniumchlorid, 2,5 g Silvet Y-12867, | |
| | 2,5 g Perfluorhexylpolyethylenglykolmethacrylat, | |
| | 10 g Polyethylenglykoldimethacrylat, | |
| | 4 g Poly[N-Vinylcaprolactam] | |
| 61 | 10 g AMPS, 20 g Acrylamid, 30 g N-2-Vinylpyrrolidon, | 3 |
| | 20 g Silvet 7608, 10 g Methacryloxypyl dimethicon, | |
| | 10 g Fluowet AC 812 | |
| 62 | 60 g AMPS, 10 g DADMAC, 10 g Quat, 10 g Genapol-LA- | 1 |
| | 250-crotonat, 10 g Methacryloxypyldimethicon, | |
| | 7 g Poly[acrylsäure-co-N-vinylformamid] | |
| 63 | 50 g AMPS, 45 g Silvet 7608, 1,8 g TMPTA, | 1 |
| | 8 g Poly[N-Vinylformamid] | |
| 64 | 20 g AMPS, 10 g Genapol T 110, 35 g MAA, 30 g | 4 |
| | HEMA, 5 g DADMAC | |
| 65 | 20 g AMPS, 80 g BB10, 1,4 g TMPTA | 1 |
| 66 | 75 g AMPS, 20 g BB10, 6,7 g Quat, 1,4 g TMPTA | 1 |
| 67 | 35 g AMPS, 60 g Acrylamid, 2 g VIFA, | 4 |
| | 2,5 g Vinylphosphonsäure, 2 Mol-% Fluowet EA-600 | |

### Chemische Bezeichnung der Reaktanden:

- AMPS: Acryloyldimethyltaurat, wahlweise Na- oder NH4-Salz
- Genapol^{®} T-080: C₁₆-C₁₈-Fettalkoholpolyglykolether mit 8 EO-Einheiten
- Genapol^{®} T-110: C₁₂-C₁₄-Fettalkoholpolyglykolether mit 11 EO-Einheiten
- Genapol^{®} T-250: C₁₆-C₁₈-Feftalkoholpolyglycolether mit 25 EO-Einheiten
- Genapol^{®} LA-040: C₁₂-C₁₄-Fettalkoholpolyglykolether mit 4 EO-Einheiten
- Genapol^{®} LA-070: C₁₂-C₁₄-Fettalkoholpolyglykolether mit 7 EO-Einheiten
- Genapol^{®} O-150 methacrylat: C₁₆-C₁₈-Fettalkoholpolyglykolether methacrylat mit 15 EO-Einheiten,
- Genapol^{®} LA-250 crotonat: C₁₂-C₁₄-Fettalkoholpolyglykolether crotonat mit 25 EO-Einheiten
- Genapol^{®} T-250 methacrylat: C₁₆-C₁₈-Fettalkoholpolyglycolether methacrylat mit 25 EO-Einheiten
- Genapol^{®} T-250 acrylat: C₁₆-C₁₈-Fettalkoholpolyglycolether methacrylat mit 25 EO-Einheiten
- BB10^{®}: Polyoxyethylen(10)Behenylether
- TMPTA: Trimethylolpropantriacrylat
- Poly-NVP: Poly-N-Vinylpyrrolidon
- Silvet^{®} 867: Siloxan Polyalkylenoxid Copolymer
- MBA: Methylen-bis-acrylamid
- AMA: Allylmethacrylat
- ^{®}Y-12867: Siloxan Polyalkylenoxid Copolymer
- Silvet^{®} 7608: Polyalkylenoxid-modifiziertes Heptamethyltrisiloxan
- Silvet^{®} 7280: Polyalkylenoxid-modifiziertes Heptamethyltrisiloxan
- DADMAC: Diallyldimethyl-ammoniumchlorid
- HEMA: 2-Hydroxyethylmethacrylat
- Quat: 2-(Methacryloyloxy)ethyltrimethylammoniumchforid
- Fluowet^{®} AC 600: Perfluoralkylethylacrylat
- Span^{®} 80: Sorbitanester

In einer bevorzugten Ausführungsform sind die Copolymere wasserlöslich oder wasserquellbar.

Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbaren Lichts liegt und deshalb Ursache der Opaleszenz sind. Durch das beschriebene, optional durchführbare Pfropfverfahren wird die Bildung übervernetzter Polymeranteile gegenüber herkömmlichen Techniken deutlich reduziert oder gänzlich vermieden.

Die beschriebene, optional durchführbare Inkorporation sowohl von kationischen Ladungen als auch von Silizium-, Fluor oder Phosphoratomen in die Copolymere führt zu Produkten, die in kosmetischen Formulierungen besondere sensorische und rheologische Eigenschaften besitzen. Eine Verbesserung der sensorischen und rheologischen Eigenschaften kann insbesondere bei der Verwendung in Rinse-off Produkten vorteilhaft sein.

Siliziummodifizierte Copolymere können die Funktionen von Silikonölen in teilweise oder in vollem Umfang übernehmen. Der Einsatz von Silikonen kann durch die Copolymere reduziert oder vermieden werden.

Die erfindungsgemäßen Deodorantien und Antiperspirantien enthalten, bezogen auf die fertigen Mittel, bevorzugt 0,01 bis 10 Gew.-%, besonders bevorzugt 0,1 bis 5 Gew.%, insbesondere bevorzugt 0,5 bis 3 Gew.-% der Copolymere.

Bevorzugt enthalten die erfindungsgemäßen Mittel 0,01 bis 89 Gew.-%, besonders bevorzugt 5 bis 50 Gew.-%, insbesondere bevorzugt 10 bis 30 Gew.-% Wasser.

Bevorzugt enthalten die erfindungsgemäßen Mittel bis 10 Gew.-%, bevorzugt 1 bis 6 Gew.-% Glycerin.
Bevorzugt enthalten die erfindungsgemäßen Mittel antimikrobielle Wirkstoffe, die die schweißzersetzenden Mikroorganismen bzw. das schweißzersetzende Esterase-Enzym hemmen.
Bevorzugt geeignet als antimikrobielle Wirkstoffe sind Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfa, Farnesol und Kombinationen dieser Wirksubstanzen.

Die erfindungsgemäßen Mittel enthalten die antimikrobiellen Mittel bevorzugt in Mengen bis 50 Gew.-%, besonders bevorzugt 0,01 bis 10 Gew.-%, insbesondere bevorzugt 0,1 bis 10 Gew.-%.

Bevorzugt enthalten die erfindungsgemäßen Mittel Adstringenzien.
Bevorzugte Adstringenzien sind Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzug von Calcium, Magnesium, Aluminium, Titan, Zirkon oder Zink.
Die erfindungsgemäßen Mittel enthalten die adstringenden Wirkstoffe bevorzugt in Mengen 0 bis 50 Gew.-%, besonders bevorzugt 0,01 bis 10 Gew.-% und insbesondere bevorzugt 0,1 bis 10 Gew.-%.

Weiterhin bevorzugt enthalten die erfindungsgemäßen Mittel Gelierungsmittel.
Als Gelierungsmittel eignen sich alle oberflächenaktiven Stoffe, die in der flüssigen Phase gelöst eine Netzwerkstruktur ausbilden und so die flüssige Phase verfestigen.

Geeignete Geliermittel sind z.B. in WO 98/58625 genannt.

Bevorzugte Gelierungsmittel sind Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Kaliumpalmitat, Aluminiummonostearat, Hydroxyfettsäuren, beispielsweise 12-Hydroxystearinsäure, -lauryl-, 16-Hydroxyhexadecanoylsäure; Fettsäureamide; Fettsäurealkanolamide; Dibenzalsorbit und alkohollösliche Polyamide und Polyacrylamide oder Mischungen solcher.

Bevorzugt enthalten die erfindungsgemäßen Mittel 0,01 bis 20 Gew.-%, besonders bevorzugt 0,1 bis 10 Gew.-%, insbesondere bevorzugt 1 bis 8 Gew.-% und ganz besonders bevorzugt 3 bis 7 Gew.-% an Geliermitteln.

In einer weiteren bevorzugten Ausführungsform enthalten die erfindungsgemäßen Mittel zusätzliche Alkohole.

Bevorzugte Alkohole sind alkoxylierte Alkohole mit bevorzugt 1 bis 80, besonders bevorzugt 3 bis 20, Alkoxygruppen und mindestens einer freien Hydroxylgruppe. Besonders bevorzugt sind Ethanol, Propanol, Isopropanol, n-Butanol, i-Butanol, t-Butanol, Glycerin, Diethylenglykol, Triethylenglykol, Dipropylenglykol, Tripropylenglykol, flüssige Polypropylenpolyethylenglycol-Copolymere, Tetrapropylenglykol, Tetraethylenglykol, Dibutylenglykol, Trimethylenglykol, Diethylenglycolmonoethylether, PEG-8, 1,3-Butandiol, 1,4-Butandiol, Glycerolpropoxylat, Propylenglycol, Hexylenglykol, 1,2-Hexandiol, 1,3-Butylenglycol, 1,2,6-Trihydroxyhexan und 1,2,3-Trihydroxhexan. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt.
Die erfindungsgemäßen Mittel enthalten bevorzugt 5 bis 90 Gew.-%, besonders bevorzugt 5 bis 80 Gew.-% und insbesondere bevorzugt 20 bis 60 Gew.-% an Alkohol.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcydohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z.B. die lonone, alpha-Isomethylionon und Methyl-cedrylketon, zu den Alkoholen Anethol, Citronellol, Eugenol, Geranion, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Die erfindungsgemäßen Mittel können als weitere Hilfs- und Zusatzstoffe Ölkörper, Emulgatoren und Co-Emulgatoren, Überfettungsmittel, feuchtigkeitsspendende Mittel, Stabilisatoren, biogene Wirkstoffe (Lokalanästhetika, Antibiotika, Antiphlogistik, Antiallergica, Corticosteroide, Sebostatika), Vitamine, Phanthenol, Allantoin, Pflanzenextrakte, beispielsweise Aloe Vera und Proteine, Glycerin, Konservierungsmittel, Perlglanzmittel, Farb- und Duftstoffe, Lösungsmittel, Hydrotrope, Enzyme, Trägersubstanzen, beispielsweise Schichtsilikate, pyrogene Kieselsäure, Elektrolytsalze wie KCl, NaCl, Komplexbildner, Antioxidation und UV-Lichtschutzfilter enthalten.

Unter Ölkörper ist jegliche Fettsubstanz zu verstehen, die bei Raumtemperatur (25°C) flüssig ist.

Die Fett-Phase kann ein oder mehrere Öle umfassen, die vorzugsweise aus folgenden Ölen ausgewählt werden:
a) Silikonöle, flüchtig oder nicht flüchtig, linear, verzweigt oder ringförmig, eventuell organisch modifiziert, Phenylsilikone, Silikonharze und -gummis, die bei Raumtemperatur fest oder flüssig sind:
b) Mineralöle, wie Paraffin- oder Vaselinöl
c) Öle tierischen Ursprungs, wie Perhydrosqualen oder Lanolin;
d) Öle pflanzlichen Ursprungs, wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl;
e) synthetische Öle, wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen, Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren,
f) Ester, wie Dioctyladipat, Diisopropyl dimer dilineloat, Propylenglykole/dicaprilat oder Wachse wie Bienenwachs, Paraffinwachs oder Mikrowachse, gegebenenfalls in Kombination mit hydrophilen Wachsen, wie z.B. Cetylstearylalkohol,
g) fluorierte und perfluorierte Öle;
h) fluorierte Silikonöle;
i) Gemische der obengenannten Substanzen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B.

Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/ oder Sorbitol zu Verfügung.

Als Stabilisatoren können Metallsalze von Fettsäuren, wie z.B. Magnesium-, Aluminium- und/oder Zinkstearat eingesetzt werden.
Unter biogenen Wirkstoffen sind beispielsweise Pflanzenextrakte und Vitaminkomplexe zu verstehen.

Die erfindungsgemäßen Mittel können mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusatz abgemischt werden.

Um die rheologischen Eigenschaften von wässrigen oder lösungsmittelhaltigen Emulsionen oder Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen, einschließlich ihrer verschiedenen Salze und Ester. Diese Polymere können wahlweise vernetzt oder unvernetzt sein.

Als UV-Filter eignen sich z.B. 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxyzimtsäure-2-ethyl-hexylester; ethoxyliertes Ethyl-4-amino-benzoat; 4-Methoxyzimtsäure-isoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)pheriol; 4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Als Antioxidantien stehen beispielsweise Superoxid-Dismutase, Tocopherol (Vitamin E) und Ascorbinsäure (Vitamin C) zur Verfügung.

Als Konservierungsmittel in Betracht kommen beispielsweise Phenoxyethanol, Parabene, Pentandiol oder Sorbinsäure.

Als Farbstoffe können die für kosmetische Zwecke geeigneten und zugelassenen Substanzen verwendet werden.

Bevorzugt handelt es sich bei den Deodorantien und Antiperspirantien um Lotionen, Cremes, Stifte (auch Mehrphasenstifte), Sprays, Roll-On-Präparate und Puder.

Die erfindungsgemäßen Mittel haben bevorzugt einen pH-Wert von 2 bis 12, besonders bevorzugt von 3 bis 8.
Die nachfolgenden Beispiele dienen der Erläuterung der Erfindung, ohne sie jedoch einzuschränken (bei allen Prozentangaben handelt es sich um Gew.-%). Bei den in den Beispielen verwendeten Copolymeren handelt es sich um Vertreter der in der Beschreibung bereits aufgeführten besonders bevorzugten Copolymere Nr.1 bis Nr.67. Die Herstellung erfolgte nach den dort angegebenen Verfahren 1, 2, 3 oder 4 unter Verwendung der bevorzugten Initiatoren und Lösemittel.

### Beispiel 1: Antiperspirant-Creme

| | | |
|---|---|---|
| A | Cithrol GMS A/S | 15,0 % |
| | Glyceryl Stearat/ PEG-100 Stearat | |
| | Crodacol C90 EP | 5,0 % |
| | Cetylalkohol | |
| | Copolymer Nr. 61 | 2,0% |
| B | Wasser | ad 100 % |
| | Sorbit (70 %ig) | 3,0% |
| | Sorbitol | |
| | Reach 501 Lsg. | 44,5 % |
| | Aluminium Chlorohydrat | |
| C | Parfümöl | q.s. |
| | Farbstoffe | q.s. |
| | Duftstoffe | q.s. |

### Herstellung:

I A und B ohne Reach 501 auf 70 bis 75°C erhitzen
II Unter Rühren abkühlen lassen
III Reach 501 bei 50 bis 55°C hinzufügen
IV Parfümöl bei 40 bis 45°C hinzugeben
V Bei 30 bis 35°C homogenisieren

### Beispiel 2: Klares Antiperspirant-Gel

| | | |
|---|---|---|
| A | Abil EM97 | 2,3 % |
| | Dimethicon Copolyl/Cyclopentasiloxan | |
| | Abil B8839 | 6,9 % |
| | Cyclopentasiloxan/Cyclohexasiloxan | |
| | Abil K4 | 6,9 % |
| | Cyclotetrasiloxan/Cyclopentasiloxan | |
| | Copolymer Nr. 28 | 1,5 % |
| B | Aloxicoll L | 40,0 % |
| | Aluminium Chlorohydrat | |
| | Propylenglykol | 25,0 % |
| | Wasser dest. | ad 100 % |
| | Parfümöl | q.s. |

### Herstellung:

I Komponenten A und B separat mischen
II Phase B langsam unter Rühren bei Raumtemperatur zur Phase A geben.
III Homogenisieren

### Beispiel 3: W/O-Antiperspirant Creme (Vergleichsbeispiel)

| | | |
|---|---|---|
| A | Abil EM90 | 2,0 % |
| | Cetyldimethicon/Copolyol | |
| | Abil B 8839 | 20,0 % |
| | Cyclopentasiloxan/Cyclohexasiloxan | |
| | Copolymer Nr. 65 | 2,0 % |
| B | Aloxicoll L | 17,0 |
| | Aluminium Chlorohydrat | |
| | Wasser dest. | ad 100 % |
| | Parfümöl | q.s. |
| | Konservierungsmittel | q.s. |

### Herstellung:

I Phase B langsam unter Rühren bei Raumtemperatur zur Phase A geben.
II Homogenisieren

### Beispiel 4: O/W-Deodorant Lotion

| | | |
|---|---|---|
| A | Teginacid H | 3,0 % |
| | Glycerylstearat/Ceteth-20 | |
| | Tegosoft, fl. | 3,0 % |
| | Cetearyl Octanoat | |
| | Tegosoft CT | 3,0 % |
| | Caprylic-/Caprictriglycerid | |
| | Copolymer Nr. 30 | 0,75 % |
| B | Glycerin | 3,0 % |
| | Wasser | ad 100 |
| C | Zitronensäure | 0,15 % |
| | Konservierungsmittel | q.s. |
| | Parfümöl | q.s. |

### Herstellung:

I Phase A und B separat auf ca. 80°C erhitzen
II Phase A und Phase B zusammenführen
III Homogenisieren
IV Unter leichtem Rühren auf 30°C abkühlen
V Phase C unterhalb von 40°C dazugeben

### Beispiel 5: Alkoholfreier Deodorant-Roll-on (opak) (Vergleichsbeispiel)

| | | |
|---|---|---|
| A | Tegodeo CW 90 | 2,0 % |
| | Zinkricinoleat/Tetrahydroxypropyl- | |
| | Ethylendiamin/Laureth-3/Propylenglykol | |
| | Polyethylenglykol(3)laurylether | 1,0 % |
| | Triethanolamin | 1,0 % |
| B | Copolymer Nr. 18 | 1,2 % |
| | Wasser dest. | ad 100 |
| C | Tagat R 40 | 3,0 % |
| | PEG-40 hydrogeniertes Castor oil | |
| | Parfümöl | q.s. |
| | Konservierungsmittel | q.s. |
| D | Zitronensäure (50%ig in Wasser) | 0,2 % |

Herstellung
I Phasen A und B separat auf 80°C erwärmen
II Phase B in Phase A einrühren und homogenisieren
III Unter langsamem Rühren abkühlen
IV Bei 30°C Phase C hinzufügen
V Den pH-Wert mit Hilfe von Phase D einstellen

### Beispiel 6: Deo-Roll-on (Vergleichsbeispiel)

| | | |
|---|---|---|
| A | Copolymer Nr. 27 | 1,0 % |
| | Wasser dest. | ad 100 |
| B | Hydagen DCMF | 0,1 |
| | Chitosan | |
| | Glykolsäure | 1,5 % |
| | Glycerin (86%) | 2,0 % |

### Herstellung:

I Copolymer Nr. 27 unter starkem Rühren langsam in das Wasser geben, bis eine klare viskose Quellung entstanden ist.
II Komponenten der Phase B unter langsamem Rühren lösen pH 4 bis 5.
III Phase B in Phase A geben und homogen rühren

### Beispiel 7: Deo-Emulsion

| | | |
|---|---|---|
| A | Eumulgin B2 Flakes | 1,0 % |
| | Ceteareth-20 | |
| | Copolymer Nr. 35 | 1,0 % |
| | Cetiol 5 | 5,0 % |
| | Dioctylcyclohexane | |
| | Cetiol OE | 5,0 % |
| | Dicaprylicether | |
| | Hydagen C.A.T. | 2,0 % |
| | Triethylcitrat | |
| B | Hydagen DCMF | 0,1 % |
| | Chitosan | |
| | Glykolsäure | 0,04 % |
| | Wasser dest. | Ad 100 |
| | Konservierungsmittel | 2,0 % |

### Herstellung:

I Die Komponenten der Phase A bei 80°C aufschmelze und homogenisieren
II Komponenten der Phase B unter Rühren lösen, und langsam unter Rühren zu A geben
III Unter Rühren abkühlen
IV Bei 30°C Konservierungsmittel zugeben

### Beispiel 8: Kristallklares alkoholfreies Gel

| | | |
|---|---|---|
| A | DC 3225 C | 10,00 % |
| | Cyclomethicon/Dimethicon Copolyol | |
| | DC 245 Fluid | 3,40 % |
| | Cyclomethicon | |
| | Polydimethylsiloxan | 3,9 % |
| | Dimethicon | |
| | Copolymer Nr. 53 | 0,75 % |
| | Parfüm 52847 | q.s |
| B | Rezal 36 G | 44,0% |
| | Aluminum Zirconium Tetrachlorohydrex GLY | |
| | Dipropylenglykol | 20 % |
| | Wasser dest. | ad 100 |

### Herstellung:

I Komponenten A und B separat gut mischen
II B zu A hinzufügen
III homogenisieren

### Beispiel 9: O/W-Antiperspirant-Creme

| | | |
|---|---|---|
| A | Copolymer Nr. 63 | 2,0% |
| | Arlamol ISML | 2,0 % |
| | Isosorbidlaurat | |
| | Dow Corning 245 Fluid | 2,0 % |
| | Cyclomethicon | |
| B | Wasser dest. | ad 100 % |
| | Locron P (50 %ig) | 40,0 % |
| | Aluminium Chlorohydrat | |
| | Konservierungsmittel | q.s |
| | Parfüm | q.s. |

### Herstellung

I Die Komponenten von A mischen
II Unter Rühren B zu A geben

## Patentansprüche

1. Deodorantien und Antiperspirantien, **dadurch gekennzeichnet, dass** sie mindestens ein Copolymer, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vemetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) einer oder mehreren mindestens monofunktioriellen, zur radikalischen Polymerisation befähigten, siliziumhaltigen Komponente(n), wobei mindestens eine siliziumhaltige Komponente eine Verbindung der Formel (I)
R¹-Z-[(Si(R³R⁴)-O-)_{w}-(Si(R⁵R⁶)-O)ₓ-]-R² (I)
ist, wobei
R¹ einen Vinyl-, Allyl-, Methallyl-, Methylvinyl-, Acryl-, Methacryl-, Crotonyl-, Senecionyl-, Itaconyl-, Maleinyl-, Fumaryl- oder ein Styrylrest darstellt;
Z eine chemische Brücke, bevorzugt ausgewählt aus -O-, -((C₁ - C₅₀) Alkylen)-, -((C₆ - C₃₀) Arylen)-, -((C₅ - C₈) Cycloalkylen)-, -((C₁-C₅₀) Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann, -((C₁ - C₁₀) Alkyl)-(Si(OCH₃)₂)- und -(Si(OCH₃)₂)-, darstellt;
R³, R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen ungesättigten, aliphatischen, cycloaliphatischen, arylaliphatischen oder aromatischen Rest mit jeweils 1 bis 50 C-Atomen oder eine Gruppe -Z-R¹ bedeutet, wobei Z und R¹ die obengenannten Bedeutungen haben,
E) gegebenenfalls einer oder mehreren mindestens monofunktionellen, zur radikalischen Polymerisation befähigten, fluorhaltigen Komponente(n),
F) gegebenenfalls einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt,
G) wobei die Copolymerisation gegebenenfalls in Gegenwart mindestens eines polymeren Additivs mit zahlenmittleren Molekulargewichten von 200 g/mol bis 10⁹ g/mol erfolgt,
enthalten.

2. Deodorantien und Antiperspirantien nach Anspruch 1, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atonien, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat, Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

3. Deodorantien und Antiperspirantien nach Anspruch 1 und/oder 2, **dadurch gekennzeichnet, dass** es sich beiden Comonomeren C) um Diallyldimethylammoniumchlorid (DADMAC),
[2-(Methacryloyloxy)ethyl]trimethylammoniurnchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchforid.
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain handelt.

4. Mittel nach mindestens einem der Ansprüche 1 bis 3, **dadurch gekennzeichnet, dass** es sich bei den fluorhaltigen Komponenten E) um Verbindungen der Formel (II)
R¹-Y-CᵣH₂ᵣCₛF₂ₛCF₃ (II)
handelt, wobei
R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine chemische Brücke, bevorzugt -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten, darstellt und
r, s stöchiometrische Koeffizienten darstellen, die unabhängig voneinander Zahlen zwischen 0 und 200 sind.

5. Deodorantien und Antiperspirantien nach mindestens einem der Ansprüche 1 bis 4, **dadurch gekennzeichnet, dass** es sich bei den Makromonömeren F) um Verbindungen der Formel (III) handelt,
R¹-Y-[(A)ᵥ-(B)_{w}-(C)ₓ-(D)_{z}]-R² (III)
wobei R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine verbrückende Gruppe, bevorzugt -O-, -S-, -C(O)-, -C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- und -N(CH₃)- darstellt;
A, B, C und D unabhängig voneinander diskrete chemische Wiederholungseinheiten, bevorzugt hervorgegangen aus Acrylamid, Methacrylamid, Ethylenoxid, Propylenoxid, AMPS, Acrylsäure, Methacrylsäure, Methylmethacrylat, Acrylnitril, Maleinsäure, Vinylacetat, Styrol, 1,3-Butadien, Isopren, Isobuten, Diethylacrylamid und Diisopropylacrylamid, insbesondere bevorzugt Ethylenoxid, Propylenoxid darstellen;
v, w, x und z unabhängig voneinander 0 bis 500, bevorzugt 1 bis 30, betragen, wobei die Summe aus v, w, x und z im Mittel ≥1 ist; und
R² einen linearen oder verzweigten aliphatischen, olefinischen, cycloaliphatischen, arylaliphatischen oder aromatischen (C₁-C₅₀)-Kohlenwasserstoffrest, OH, -NH₂ oder -N(CH₃)₂ darstellt oder gleich [-Y-R¹] ist.

6. Deodorantien und Antiperspirantien nach mindestens einem der Ansprüche 1 bis 5, **dadurch gekennzeichnet, dass** es sich bei den polymeren Additiven G) um Homo- oder Copolymere aus N-Vinylformamid, N-Vinylacetamid, N-Vinylpyrrolidon, Ethylenoxid, Propylenoxid, Acryloyldirnethyltaurinsäure, N-Vinylcaprolactam, N-Vinytmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimeihylammoniumchlorid (MAPTAC); Polyalkylenglykole und/oder Alkylpolyglykole handelt.

7. Deodorantien und Antiperspirantien nach mindestens einem der Ansprüche 1 bis 6, **dadurch gekennzeichnet, dass** die Copolymerisation in Gegenwart mindestens eines polymeren Additivs G) erfolgt.

8. Deodorantien und Antiperspirantien nach mindestens einem der Ansprüche 1 bis 7, **dadurch gekennzeichnet, dass** die Copolymere vernetzt sind.

9. Deodorantien und Antiperspirantien nach mindestens einem der Ansprüche 1 bis 8, **dadurch gekennzeichnet, dass** die Copolymere durch Fällungspolymerisation in tert.-Butanol hergestellt werden.

10. Deodorantien und Antiperspirantien nach mindestens einem der Ansprüche 1 bis 9, **dadurch gekennzeichnet, dass** die Copolymere wasserlöslich oder wasserquellbar sind.

11. Deodorantien und Antiperspiranfien nach mindestens einem der Ansprüche 1 bis 10, **dadurch gekennzeichnet, dass** sie, bezogen auf die fertigen Mittel, 0,01 bis 10 Gew.-% der Copolymere enthalten.

12. Deodorantien und Antiperspirantien nach mindestens einem der Ansprüche 1 bis 11, **dadurch gekennzeichnet, dass** sie 0,01 bis 89 Gew.-% Wasser enthalten.

13. Deodorantien und Antiperspirantien nach mindestens einem der Ansprüche 1 bis 12, **dadurch gekennzeichnet, dass** sie bis 10 Gew.-% Glycerin enthalten.

14. Deodorantien und Antiperspirantien nach mindestens einem der Ansprüche 1 bis 13, **dadurch gekennzeichnet, dass** sie antimikrobielle Wirkstoffe enthalten.

15. Deodorantien und Antiperspirantien nach mindestens einem der Ansprüche 1 bis 14, **dadurch gekennzeichnet, dass** sie Adstringenzien enthalten.

16. Deodorantien und Antiperspirantien nach mindestens einem der Ansprüche 1 bis 15, **dadurch gekennzeichnet, dass** es sich dabei um Lotionen, Cremes, Stifte, Mehrphasenstifte, Sprays, Roll-On-Präparate oder Puder handelt.

17. Deodorantien und Antiperspirantien nach mindestens einem der Ansprüche 1 bis 16, **dadurch gekennzeichnet, dass** mindestens eine siliziumhaltige . Komponente eine Verbindung ausgewählt aus den Formeln 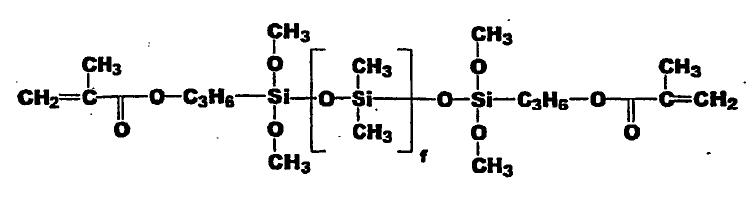 worin f = 2 bis 500 bedeutet, 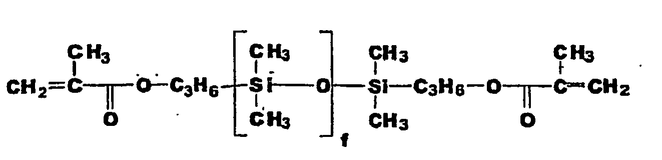 worin f = 2 bis 500 bedeutet, und 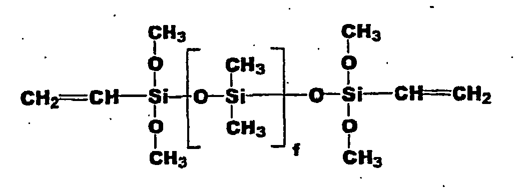 worin f = 2 - 500 bedeutet, ist.

## Claims

1. A deodorant or antiperspirant which comprises at least one copolymer obtainable by free-radical copolymerization of
A) acryloyldimethyltaurine and/or acryloyldimethyltaurates,
B) if desired, one or more further olefinically unsaturated, noncationic, optionally crosslinking comonomers which have at least one oxygen, nitrogen, sulfur or phosphorus atom and possess a molecular weight of less than 500 g/mol,
C) if desired, one or more olefinically unsaturated, cationic comonomers which have at least one oxygen, nitrogen, sulfur or phosphorus atom and possess a molecular weight of less than 500 g/mol,
D) one or more silicon-containing components capable of free-radical polymerization and having a functionality of at least one, at least one silicon-containing component being a compound of the formula (I)
R¹-Z-[(Si(R³R⁴)-O-)_{w}-(Si(R⁵R⁶)-O)ₓ-]-R² (I)
where
R¹ represents a vinyl, allyl, methallyl, methylvinyl, acryloyl, methacryloyl, crotonyl, senecionyl, itaconyl, maleyl, fumaryl or styryl radical;
Z is a chemical bridge, preferably -O-, -((C₁-C₅₀)alkylene)-, - ((C₆-C₃₀) arylene) -, - ((C₅-C₈) cycloalkylene) -, -((C₁-C₅₀)alkenylene) -, -(polypropylene oxide)ₙ-, -(polyethylene oxide)ₒ-, -(polypropylene oxide)ₙ(polyethylene oxide)ₒ-, where n and o independently of one another denote numbers from 0 to 200, and the distribution of the EO/PO units can be random or in the form of blocks, -((C₁-C₁₀)alkyl)-(Si(OCH₃)₂)- and -(Si(OCH₃)₂)-;
R³, R⁴, R⁵, and R⁶ independently of one another are -CH₃, -O-CH₃, -C₆H₅ or -O-C₆H₅;
w, x denote numbers from 0 to 500, it being necessary for either w or x to be greater than zero, and
R² is an unsaturated aliphatic, cycloaliphatic, arylaliphatic or aromatic radical having in each case 1 to 50 carbon atoms or a group -Z-R¹, where Z and R¹ have the abovementioned definitions,
E) if desired, one or more fluorine-containing components capable of free-radical polymerization and having a functionality of at least one,
F) if desired, one or more olefinically mono- or polyunsaturated, optionally crosslinking macromonomers each possessing at least one oxygen, nitrogen, sulfur or phosphorus atom and having a number-average molecular weight of greater than or equal to 200 g/mol, the macromonomers not being a silicon-containing component D) or fluorine-containing component E),
G) the copolymerization taking place if desired in the presence of at least one polymeric additive having number-average molecular weights of from 200 g/mol to 10⁹ g/mol.

2. A deodorant or antiperspirant as claimed in claim 1, wherein the comonomers B) are unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate, styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

3. A deodorant or antiperspirant as claimed in claim 1 and/or 2, wherein the comonomers C) are diallyldimethylammonium chloride (DADMAC),
[2-(methacryloyloxy)ethyl]trimethylammonium chloride (MAPTAC),
[2-(acryloyloxy)ethyl]trimethylammonium chloride,
[2-methacrylamidoethyl]trimethylammonium chloride,
[2-(acrylamido)ethyl]trimethylammonium chloride,
N-methyl-2-vinylpyridinium chloride,
N-methyl-4-vinylpyridinium chloride,
dimethylaminoethyl methacrylate,
dimethylaminopropylmethacrylamide,
methacryloylethyl N-oxide and/or
methacryloylethylbetaine.

4. A composition as claimed in at least one of claims 1 to 3, wherein the fluorine-containing components E) are compounds of the formula (II)
R¹-Y-CᵣH₂ᵣCₛF₂ₛCF₃ (II)
where
R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a chemical bridge, preferably -O-, -C(O)-,-C(O)-O-, -S-, -O-CH₂-CH(O-)-CH₂OH, -O-CH₂-CH(OH)-CH₂-O-, -O-SO₂-O--O-S(O)-O-, -PH-, -P(CH₃)-, -PO₃-, -NH-, -N(CH₃)-, -O- (C₁-C₅₀)alkyl-O-, -O-phenyl-O-, -O-benzyl-O-, -O-(C₅-C₈)cycloalkyl-O--O-(C₁-C₅₀)alkenyl-O-, -O-(CH(CH₃)-CH₂-O)ₙ-, -O-(CH₂-CH₂-O)ₙ-, and -O-([CH-CH₂-O]ₙ-[CH₂-CH₂-O]ₘ)ₒ-, where n, m, and o independently of one another denote numbers from 0 to 200, and
r, s are stoichiometric coefficients which independently of one another are numbers between 0 and 200.

5. A deodorant or antiperspirant as claimed in at least one of claims 1 to 4, wherein the macromonomers F) are compounds of the formula (III)
R¹-Y-[(A)ᵥ-(B)_{w}-(C)ₓ-(D)_{z}]-R² (III)
where R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a bridging group, preferably -O-, -S-, -C(O)-, - C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH-, and -N(CH₃)-;
A, B, C, and D independently of one another are discrete chemical repeating units, preferably originating from acrylamide, methacrylamide, ethylene oxide, propylene oxide, AMPS, acrylic acid, methacrylic acid, methyl methacrylate, acrylonitrile, maleic acid, vinyl acetate, styrene, 1,3-butadiene, isoprene, isobutene, diethylacrylamide, and diisopropylacrylamide, more preferably ethylene oxide or propylene oxide;
v, w, x, and z independently of one another amount to from 0 to 500, preferably from 1 to 30, the sum of v, w, x, and z being on average ≥ 1; and
R² is a linear or branched aliphatic, olefinic, cycloaliphatic, arylaliphatic or aromatic (C₁-C₅₀) hydrocarbon radical, OH, -NH₂ or -N(CH₃)₂ or is [-Y-R¹].

6. A deodorant or antiperspirant as claimed in at least one of claims 1 to 5, wherein the polymeric additives G) are homopolymers or copolymers of N-vinylformamide, N-vinylacetamide, N-vinylpyrrolidone, ethylene oxide, propylene oxide, acryloyldimethyltaurine, N-vinylcaprolactam, N-vinylmethylacetamide, acrylamide, acrylic acid, methacrylic acid, N-vinylmorpholide, hydroxymethyl methacrylate, diallyldimethylammonium chloride (DADMAC) and/or [2-(methacryloyloxy)ethyl]trimethylammonium chloride (MAPTAC); polyalkylene glycols and/or alkylpolyglycols.

7. A deodorant or antiperspirant as claimed in at least one of claims 1 to 6, wherein the copolymerization takes place in the presence of at least one polymeric additive G).

8. A deodorant or antiperspirant as claimed in at least one of claims 1 to 7, wherein the copolymers are crosslinked.

9. A deodorant or antiperspirant as claimed in at least one of claims 1 to 8, wherein the copolymers are prepared by precipitation polymerization in tert-butanol.

10. A deodorant or antiperspirant as claimed in at least one of claims 1 to 9, wherein the copolymers are water-soluble or water-swellable.

11. A deodorant or antiperspirant as claimed in at least one of claims 1 to 10, which comprises, based on the finished composition, from 0.01 to 10% by weight of the copolymers.

12. A deodorant or antiperspirant as claimed in at least one of claims 1 to 11, which contains from 0.01 to 89% by weight of water.

13. A deodorant or antiperspirant as claimed in at least one of claims 1 to 12, which contains up to 10% by weight of glycerol.

14. A deodorant or antiperspirant as claimed in at least one of claims 1 to 13, which comprises active antimicrobial substances.

15. A deodorant or antiperspirant as claimed in at least one of claims 1 to 14, which comprises astringents.

16. A deodorant or antiperspirant as claimed in at least one of claims 1 to 15, which is in the form of a lotion, cream, stick, multiphase stick, spray, roll-on preparation or powder.

17. A deodorant or antiperspirant as claimed in at least one of claims 1 to 16, wherein at least one silicon-containing component is a compound selected from the formulae 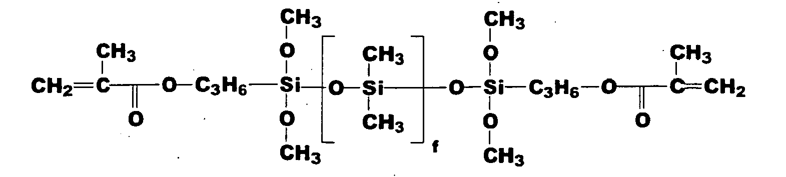 in which f = 2 to 500, 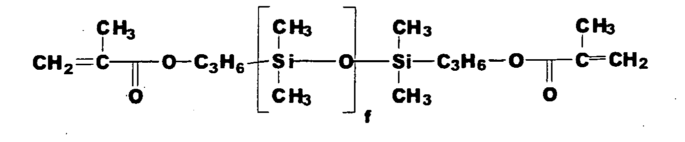 in which f = 2 to 500, and 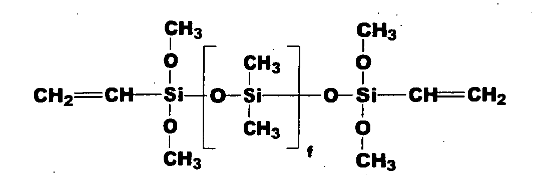 in which f = 2-500.

## Revendications

1. Déodorants et antitranspirants, **caractérisés en ce qu'**ils contiennent au moins un copolymère pouvant être obtenu au moyen d'une copolymérisation radicalaire de
A) acide acryloyldiméthyltaurique et/ou acryloyldiméthyltaurates,
B) éventuellement un ou plusieurs autres comonomères à insaturation oléfinique, non cationiques, éventuellement réticulants, qui présentent au moins un atome d'oxygène, d'azote, de soufre ou de phosphore et possèdent une masse moléculaire inférieure à 500 g/mol,
C) éventuellement un ou plusieurs comonomères à insaturation oléfinique, cationiques, qui présentent au moins un atome d'oxygène, d'azote, de soufre ou de phosphore et possèdent une masse moléculaire inférieure à 500 g/mol,
D) un ou plusieurs composants au moins monofonctionnels contenant du silicium, polymérisables par voie radicalaire, parmi lesquels au moins un composant contenant du silicium est un composé de formule (I)
R¹-Z-[(Si(R³R⁴)-O-_{w}-(Si(R⁵R⁶)-O)ₓ-]-R² (I)
dans laquelle
R¹ représente un groupe vinyle, allyle, méthallyle, méthylvinyle, acryle, méthacryle, crotonyle, sénécionyle, itaconyle, maléinyle, fumaryle ou styryle ;
Z représente un pont chimique, choisi de préférence parmi -O-, -(alkylène en C₁ à C₅₀)-, -(arylène en C₆ à C₃₀)-, -(cycloalkylène en C₅ à C₈)-, -(alcénylène en C₁ à C₅₀)-, -(poly(oxyde de propylène))ₙ-, -(poly(oxyde d'éthylène))ₒ-, -(poly(oxyde de propylène))ₙ(poly(oxyde d'éthylène))ₒ-, où n et o représentent chacun, indépendamment l'un de l'autre, un nombre de 0 à 200 et la distribution des motifs OE/OP peut être statistique ou séquencée, un groupe -(alkyle en C₁ à C₁₀)-(Si(OCH₃)₂)- et -(Si(OCH₃)₂)- ;
R³, R⁴, R⁵ et R⁶ représentent, indépendamment les uns des autres, -CH₃, -O-CH₃, -C₆H₅ ou -O-C₆H₅ ;
w, x représentent un nombre de 0 à 500, w ou x devant être supérieur à zéro, et
R² représente un groupe insaturé, aliphatique, cycloaliphatique, arylaliphatique ou aromatique contenant 1 à 50 atomes de carbone ou un groupe -Z-R¹, où Z et R¹ ont les significations susmentionnées,
E) éventuellement un ou plusieurs composants au moins monofonctionnels contenant du fluor, polymérisables par voie radicalaire,
F) éventuellement un ou plusieurs macromonomères à mono- ou poly-insaturation oléfinique, éventuellement réticulants, qui présentent chacun au moins un atome d'oxygène, d'azote, de soufre ou de phosphore et possèdent une masse moléculaire moyenne en nombre supérieure ou égale à 200 g/mol, les macromonomères n'étant pas un composant contenant du silicium D) ou un composant contenant du fluor E),
G) où la copolymérisation est éventuellement réalisée en présence d'au moins un additif polymère ayant une masse moléculaire moyenne en nombre de 200 g/mol à 10⁹ g/mol.

2. Déodorants et antitranspirants selon la revendication 1, **caractérisés en ce que** les comonomères B) sont des acides carboxyliques insaturés, des sels d'acides carboxyliques insaturés, des anhydrides d'acides carboxyliques insaturés, des esters d'acides carboxyliques insaturés avec des alcools aliphatiques, oléfiniques, cycloaliphatiques, arylaliphatiques ou aromatiques contenant 1 à 22 atomes de carbone, des N-vinylamides à chaîne ouverte, des N-vinylamides cycliques ayant une taille de cycle de 3 à 9, des amides de l'acide acrylique, des amides de l'acide méthacrylique, des amides d'acides acryliques substitués, des amides d'acides méthacryliques substitués, la 2-vinylpyridine, la 4-vinylpyridine, l'acétate de vinyle, le styrène, l'acrylonitrile, le chlorure de vinyle, le chlorure de vinylidène, le tétrafluoroéthylène, l'acide vinylphosphonique ou ses esters ou sels, l'acide vinylsulfonique ou ses esters ou sels, l'acide allylphosphonique ou ses esters ou sels et/ou l'acide méthallylsulfonique ou ses esters ou sels.

3. Déodorants et antitranspirants selon la revendication 1 et/ou 2, **caractérisés en ce que** les comonomères C) sont le chlorure de diallyldiméthylammonium (DADMAC),
le chlorure de [2-(méthacryloyloxy)éthyl]-triméthylammonium (MAPTAC),
le chlorure de [2-(acryloyloxy)éthyl]triméthylammonium,
le chlorure de[2-méthacrylamidoéthyl]triméthylammonium,
le chlorure de [2-(acrylamido)éthyl]triméthylammonium,
le chlorure de N-méthyl-2-vinylpyridinium,
le chlorure de N-méthyl-4-vinylpyridinium,
le diméthylaminoéthylméthacrylate,
le diméthylaminopropylméthacrylamide,
le N-oxyde de méthacryloyléthyle et/ou
la méthacryloyléthyl-bétaïne.

4. Produit selon au moins l'une quelconque des revendications 1 à 3, **caractérisé en ce que** les composants contenant du fluor E) sont des composés de formule (II)
R¹-Y-CᵣH₂ᵣCₛF₂ₛCF₃ (II)
dans laquelle
R¹ représente une fonction polymérisable du groupe des composés à insaturation vinylique, de préférence un groupe vinyle, allyle, méthallyle, méthylvinyle, acryle, méthacryle, crotonyle, sénécionyle, itaconyle, maléinyle, fumaryle ou styryle ;
Y représente un pont chimique, de préférence -O-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-(alkyle en C₁ à C₅₀)-O-, -O-phényle-O-, -O-benzyle-O-, -O-(cycloalkyle en C₅ à C₈)-O-, -O-(alcényle en C₁ à C₅₀)-O-, -O-(CH(CH₃)-CH₂-O)ₙ-, -O-(CH₂-CH₂-O)ₙ- et O-([CH-CH₂-O]ₙ-[CH₂-CH₂-O]ₘ)ₒ-, où n, m et o représentent chacun, indépendamment les uns des autres, un nombre de 0 à 200, et
r, s représentent des coefficients stoechiométriques qui sont, indépendamment l'un de l'autre, des nombres compris entre 0 et 200.

5. Déodorants et antitranspirants selon au moins l'une quelconque des revendications 1 à 4, **caractérisés en ce que** les macromonomères F) sont des composés de formule (III)
R¹-Y-[(A)_{V}-(B)_{W}-(C)_{X}-(D)_{Z}]-R² (III)
dans laquelle
R¹ représente une fonction polymérisable du groupe des composés à insaturation vinylique, de préférence un groupe vinyle, allyle, méthallyle, méthylvinyle, acryle, méthacryle, crotonyle, sénécionyle, itaconyle, maléinyle, fumaryle ou styryle ;
Y représente un groupe pontant, de préférence -O-, -S-, -C(O)-, -C(O)-O-, -O-CH₂-CH(O-)-CH₂OH, -O-CH₂-CH(OH)-CH₂O-, -O-SO₂-O-, -O-SO₂-O-, -O-SO-O-, -PH-, -P(CH₃)-, -PO₃-, -NH- et -N(CH₃)-;
A, B, C et D représentent, indépendamment les uns des autres, des motifs chimiques récurrents discrets, dérivés de préférence d'acrylamide, de méthacrylamide, d'oxyde d'éthylène, d'oxyde de propylène, d'AMPS, d'acide acrylique, d'acide méthacrylique, de méthacrylate de méthyle, d'acrylonitrile, d'acide maléique, d'acétate de vinyle, de styrène, de 1,3-butadiène, d'isoprène, d'isobutène, de diéthylacrylamide et de diisopropylacrylamide, de manière plus particulièrement préférée d'oxyde d'éthylène, d'oxyde de propylène ;
v, w, x et z représentent chacun, indépendamment les uns des autres, un nombre de 0 à 500, de préférence de 1 à 30, la somme de v, w, x et z étant en moyenne supérieure ou égale à 1 ; et
R² représente un groupe hydrocarboné en C₁ à C₅₀ aliphatique, oléfinique, cycloaliphatique, arylaliphatique ou aromatique, linéaire ou ramifié, OH, -NH₂ ou -N(CH₃)₂ ou le groupe [-Y-R¹].

6. Déodorants et antitranspirants selon au moins l'une quelconque des revendications 1 à 5, **caractérisés en ce que** les additifs polymères G) sont des homo- ou copolymères de N-vinylformamide, de N-vinylacétamide, de N-vinylpyrrolidone, d'oxyde d'éthylène, d'oxyde de propylène, d'acide acryloyldiméthyltaurique, de N-vinylcaprolactame, de N-vinylméthylacétamide, d'acrylamide, d'acide acrylique, d'acide méthacrylique, de N-vinylmorpholide, d'hydroxyméthylméthacrylate, de chlorure de diallyldiméthylammonium (DADMAC) et/ou de chlorure de [2-(méthacryloyloxy)éthyl]triméthylammonium (MAPTAC) ; des polyalkylèneglycols et/ou des alkylpolyglycols.

7. Déodorants et antitranspirants selon au moins l'une quelconque des revendications 1 à 6, **caractérisés en ce que** la copolymérisation est réalisée en présence d'au moins un additif polymère G).

8. Déodorants et antitranspirants selon au moins l'une quelconque des revendications 1 à 7, **caractérisés en ce que** les copolymères sont réticulés.

9. Déodorants et antitranspirants selon au moins l'une quelconque des revendications 1 à 8, **caractérisés en ce que** les copolymères sont préparés au moyen d'une polymérisation par précipitation dans du tert-butanol.

10. Déodorants et antitranspirants selon au moins l'une quelconque des revendications 1 à 9, **caractérisés en ce que** les copolymères sont hydrosolubles ou gonflent dans l'eau.

11. Déodorants et antitranspirants selon au moins l'une quelconque des revendications 1 à 10, **caractérisés en ce qu'**ils contiennent 0,01% à 10% en poids de copolymères par rapport aux produits finis.

12. Déodorants et antitranspirants selon au moins l'une quelconque des revendications 1 à 11, **caractérisés en ce qu'**ils contiennent 0,01% à 89% en poids d'eau.

13. Déodorants et antitranspirants selon au moins l'une quelconque des revendications 1 à 12, **caractérisés en ce qu'**ils contiennent jusqu'à 10% en poids de glycérine.

14. Déodorants et antitranspirants selon au moins l'une quelconque des revendications 1 à 13, **caractérisés en ce qu'**ils contiennent des agents antimicrobiens.

15. Déodorants et antitranspirants selon au moins l'une quelconque des revendications 1 à 14, **caractérisés en ce qu'**ils contiennent des astringents.

16. Déodorants et antitranspirants selon au moins l'une quelconque des revendications 1 à 15, **caractérisés en ce qu'**il s'agit de lotions, de crèmes, de bâtons, de bâtons à plusieurs phases, de fluides à pulvériser, de préparations pour applicateur à bille ou de poudres.

17. Déodorants et antitranspirants selon au moins l'une quelconque des revendications 1 à 16, **caractérisés en ce qu'**au moins un composant contenant du silicium est un composé choisi parmi les formules 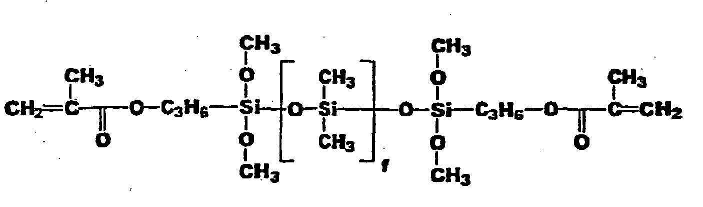 dans laquelle f vaut 2 à 500, 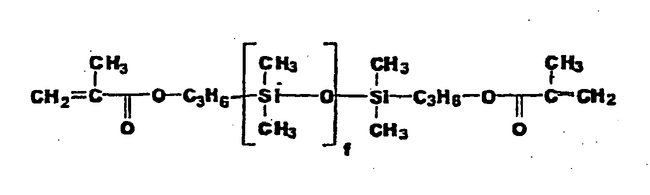 dans laquelle f vaut 2 à 500, et 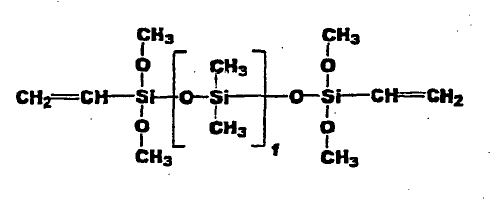 dans laquelle f vaut 2 à 500.
